# EUROPÄISCHE PATENTSCHRIFT

(11) **EP 1 580 337 B1**
(45) Veröffentlichungstag und Bekanntmachung des Hinweises auf die Patenterteilung: **18.01.2012**
(21) Anmeldenummer: 05005834.6
(22) Anmeldetag: 17.03.2005
(51) Int. Cl.: E03C 1/126, A47K 1/14, E03C 1/23

(54) **Abflußstopfen**
Drain plug
Bouchon

(30) Priorität: 26.03.2004 DE 102004014941
(43) Veröffentlichungstag der Anmeldung: 28.09.2005
(73) Patentinhaber: Bilz, Sonja Maria, 61130 Nidderau (DE)
(72) Erfinder: Bilz, Sonja Maria, 61130 Nidderau (DE)
(74) Vertreter: Tergau & Walkenhorst

(56) Entgegenhaltungen:
- EP-A- 0 409 400
- DE-A1- 10 220 996
- DE-U1- 20 103 271
- FR-A- 2 837 223
- FR-A- 2 837 224
- GB-A- 2 285 216
- US-A- 3 112 499
- US-A- 5 860 173

## Beschreibung

Die Erfindung betrifft einen Verschlussstopfen für eine Ablauföffnung eines Beckens oder Behälters und umfasst die Merkmale des Oberbegriffes von Patentanspruch 1.

Verschlussstopfen der hier interessierenden Art sind bekannt. Sie weisen ein am Rand einer Ablauföffnung eines Beckens, Behälters oder dergleichen, insbesondere eines Wasser- oder Waschbeckens, eines Spülbeckens, einer Badewanne, einer Duschwanne oder dergleichen anliegendes Verschlussteil auf und ragen dabei in der Regel etwas in die Ablauföffnung hinein.

Die FR 2 837 224 offenbart einen Verschlussstopfen mit einem eine Dichtfläche aufweisenden und am Rand einer Ablauföffnung unmittelbar anliegenden Verschlussteil, an dem ablaufseitig ein von Wasser umströmter, teilweise offener Träger vorgesehen ist, wobei der Träger eine sich allmählich im Wasser verbrauchende Substanz aufnehmen soll und dazu im Bereich der Ablauföffnung eine Gitterstruktur und somit eine Vielzahl von Durchtrittsöffnungen aufweist.

In der US 3,112,499 ist ein Verschlussstopfen mit einem eine Dichtfläche aufweisenden und am Rand einer Ablauföffnung unmittelbar anlegbaren Verschlussteil aufgezeigt, an dem ablaufseitig ein von Wasser umströmter, teilweise offener Träger vorgesehen ist, wobei der Träger eine sich allmählich im Wasser verbrauchende Substanz aufnehmen soll und dazu im Bereich der Ablauföffnung zwischen einer Anzahl von Wandrippen mehrere Durchtrittsöffnungen aufweist.

Die DE 102 20 996 beschreibt einen Verschlussstopfen für ein Abflussrohr mit einer Abflussöffnung in einem Grundkörper, der mindestens eine Dichtung und ein am Rand einer Ablassöffnung eines sanitären Elementes unmittelbar anliegendes Verschlussteil aufweist, wobei der Verschlussstopfen ein stopfenartiges und ein siebartiges Ende aufweist, wobei das stopfenartige Ende ein mit dem siebartigen Ende fest verbundenes Dichtungsteil zur umfangsdichten Festlegung in dem Abflussrohr und einen Deckelteil aufweist, welches an dem Dichtungsteil lösbar verbunden ist, und mit einer Einlassöffnung in dem Grundkörper zum Einlassen einer durch das siebartige Ende abfließenden Flüssigkeit.

Das siebartige Ende ist dabei nicht geeignet, eine sich im Wasser allmählich verbrauchende Substanz aufzunehme. Es dient nur zum Auflange von Haaren.

Die unterhalb der Ablauföffnung befindlichen Teile des Beckens bzw. Ablaufrohres oder auch des Verschlussstopfens selbst sind schwer zugänglich und lassen sich daher grundsätzlich kaum reinigen. Es bilden sich dort Ablagerungen, die häufig auch unangenehme Gerüche zur Folge haben. Der Erfindung liegt daher die Aufgabe zugrunde, Maßnahmen vorzusehen, mit deren Hilfe es möglich ist, das Entstehen von Verschmutzungen, Ablagerungen und/oder von unangenehmen Gerüchen zu vermeiden bzw. die Verschmutzungen und Ablagerungen wieder zu entfernen.

Zur Lösung dieser Aufgabe sieht die Erfindung vor, dass ablaufseitig ein von Wasser umströmter, mindestens teilweise offene Träger für eine sich im Wasser allmählich verbrauchende Substanz bzw. für ein Mittel vorgesehen ist.

Die sich verbrauchende Substanz ist ein Reinigungs-, Desinfektions- und/oder Duftstoff, der wasserlöslich ist und sich während einer längeren Zeit auflöst. Die Substanz benetzt im aufgelösten Zustand den Ablaufbereich unterhalb der Ablauföffnung und sorgt für die erwünschte Reinigung, Desinfektion bzw. Geruchsunter drückung oder sie erzeugt gezielt als angenehm empfundene Gerüche oder Dürfte.

Der Behälter weist ferner in Weiterbildung der Erfindung an seinem Außenumfang Distanz- und Führungsrippen auf. Die Durchtrittsöffnungen können kreisrund oder auch Langlöcher sein.

Weitere Merkmale der Erfindung gehen aus Unteransprüchen und der Beschreibung im Zusammenhang mit der Zeichnung hervor.

Die Erfindung wird nachstehend anhand von Ausführungsbeispielen, die in der Zeichnung dargestellt sind, näher erläutert. Dabei zeigen:
- Fig. 1: im Schnitt sowie im größeren Maßstab einen in einer Ablauföffnung eines Beckens angeordneten Verschlussstopfen;
- Fig. 2: eine perspektivische Ansicht eines abgewandelten Verschlussstop- fens schräg von unten;
- Fig. 3: in auseinander gezogener sowie perspektivischer Darstellung die Einzelteile des Verschlussstopfens nach Fig. 2 in einer Ansicht eben- falls schräg von unten;
- Fig. 4: eine Ansicht wie in Fig. 1 von einer weiteren, abgewandelten Ausfüh- rungsform;
- Fig. 5: eine Ansicht wie in den Figuren 1 und 4 in einer vierten Ausführungsform;
- Fig. 6: eine Ansicht wie in den Figuren 1 und 4 von einem fünften Ausführungsbeispiel;
- Fig. 7: im Schnitt eine Ansicht von einem sechsten Ausführungsbeispiel, und
- Fig. 8: eine Prinzipskizze von einem letzten Ausführungsbeispiel.

Ein Verschlussstopfen 1 umfasst ein Verschlussteil 2 mit mindestens einer Dichtfläche oder Dichtung 3, die am Rand 4 einer Ablauföffnung 5 eines Beckens 6 oder Behälters in der Schließstellung anliegt. Mit Hilfe des Verschlussstopfens 1 und eines höhenverstellbaren Anschlages 7 lässt sich die Ablauföffnung 5 einerseits öffnen und andererseits mehr oder weniger verschließen, so dass Wasser aus dem Becken 6 leicht oder weniger leicht bzw. gar nicht auslaufen kann.

Ablaufseitig ist an dem Verschlussstopfen 1 ein von dem ablaufenden Wasser umströmter, mindestens teilweise offener Träger 8 für eine sich mit Hilfe von Wasser verbrauchende Substanz 9/ein Mittel 9 angeordnet. Die Substanz 9 kommt mit dem ablaufenden Wasser in Kontakt und löst sich dabei langsam auf mit der Folge, dass der Bereich der Ablauföffnung 5 und alle in der Nähe bzw. darunter befindlichen Teile des Beckens 6 und auch die Teile des Verschlussstopfens 1 gereinigt, desinfiziert und/oder mit geruchshemmenden Stoffen versehen werden. Auch Duftstoffe oder dergleichen können hierbei verwendet werden.

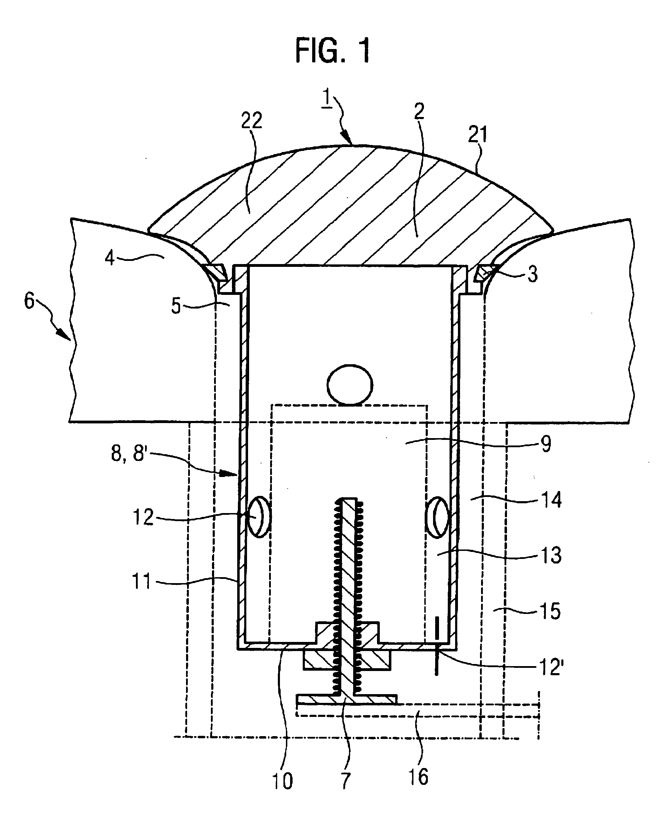

Der Träger 8 ist gemäß dem in Fig. 1 dargestellten Ausführungsbeispiel ein Behälter 8', der im Bereich seines Bodens 10 und/oder im Bereich einer Behälterwand 11 mindestens eine Durchtrittsöffnung 12 für das Wasser aufweist. Ein Flüssigkeits- und Substanzaustausch zwischen dem Behälterinneren 13 und dem Raum 14 außerhalb des Behälters 12 ist somit möglich, wobei der Raum 14 nach außen gemäß dem in Fig. 1 dargestellten Ausführungsbeispiel von einem Rohr 15 begrenzt wird.

Der höhenverstellbare Anschlag 7 stützt sich gemäß Fig. 1 z. B. auf einem Arm 16 ab, der in an sich bekannter Weise durch das Rohr 15 greift und in der Regel verstellbar ist.

Der Träger 8 bzw. Behälter 8' ist an der Unterseite des Verschlussteiles 2 in lösbarer Weise angeordnet, wozu beispielsweise eine Rast- oder Steckverbindung dient.

Das Verschlussteil 2 und der Behälter 8' bilden gemeinsam eine höhenverstellbare Einheit, wobei der Anschlag 7 z. B. am Boden 10 des Behälters 8' verstellbar, ferner mit Hilfe des Armes 16 und somit auch der Verschlussstopfen 1 selbst in verschiedene Positionen bewegbar ist.

Durchtrittsöffnungen 12' können sich im Boden 10 ebenso wie in der Behälterwand 11 befinden und z. B. kreisrund sein.

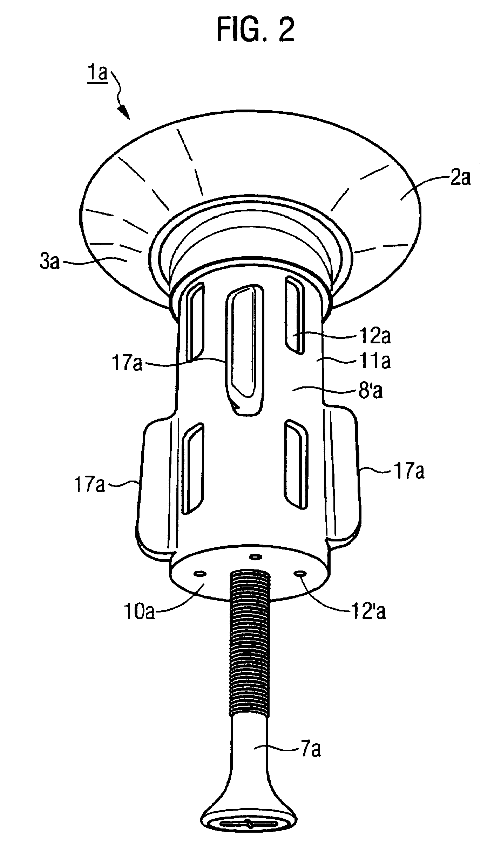

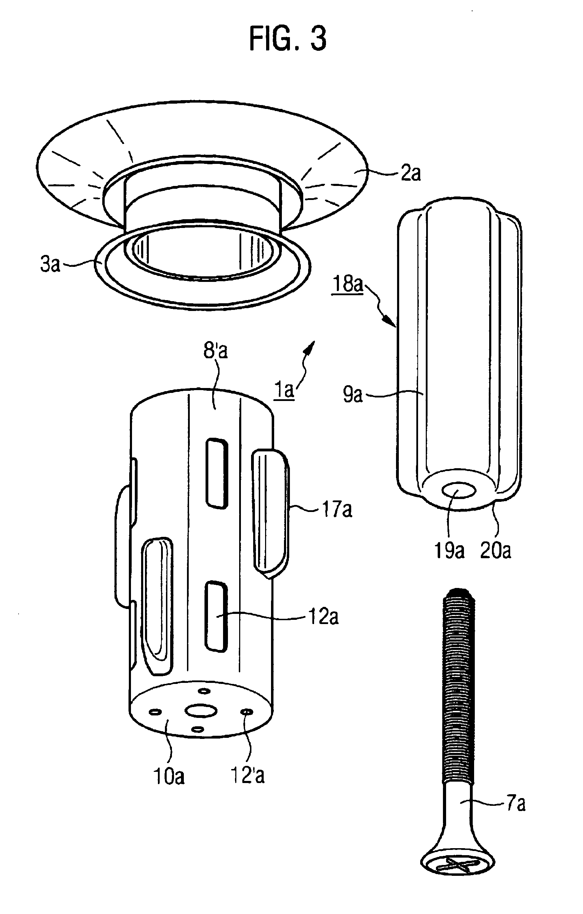

Eine abgewandelte Ausführungsform von einem Verschlussstopfen 1 a ist perspektivisch in Fig. 2 und ebenfalls perspektivisch entsprechend seinen Einzelteilen in Fig. 3 dargestellt, wobei grundsätzlich gleiche Teile dieselben Bezugszahlen und im Buchstabenindex a aufweisen.

Der Verschlussstopfen 1a umfasst ein Verschlussteil 2a, eine Dichtung 3a, einen Behälter 8'a, eine als Anschlag 7a dienende Schraube sowie eine sich allmählich verbrauchende Substanz 9a, die sich im zusammengebauten Zustand im Inneren des Behälters 8'a befindet.

Als Durchtrittsöffnungen 12a weist der Behälter 8'a in der Behälterwand 11a zweckmäßigerweise Langlöcher auf, während kreisrunde Durchtrittsöffnungen 12'a im Boden 10a angeordnet sind. Ferner sind am Außenumfang des Behälters 8'a mehrere Distanzrippen 17a vorgesehen, die für einen guten Sitz des Verschlussstopfens 1a mit seinem Behälter 8'a z. B. in einem Ablaufrohr sorgen.

Die Substanz 9, 9a ist zweckmäßigerweise ein wasserlöslicher Festkörper 18a. Auch ist es vorteilhaft, wenn dieser Festkörper 18a einen an den lichten Querschrtitt des Behälters 8', 8'a angepassten Querschnitt aufweist. Dazu gehört nicht nur, dass er eine Längsbohrung 19a zur Aufnahme von Teilen des längsverstellbaren Anschlages 7, 7a aufweist, sondern auch sein Außenumfang 20a weicht zweckmäßigerweise von einer reinen Kreisform ab. Gemäß Ausführungsbeispiel ist der Festkörper 18a außen mit wellenförmigen Vertiefungen und Erhebungen bzw. mit Rippen und ineinander übergehenden Rillen versehen. Der Festkörper weist somit im Querschnitt ein charakteristisches Profil auf.

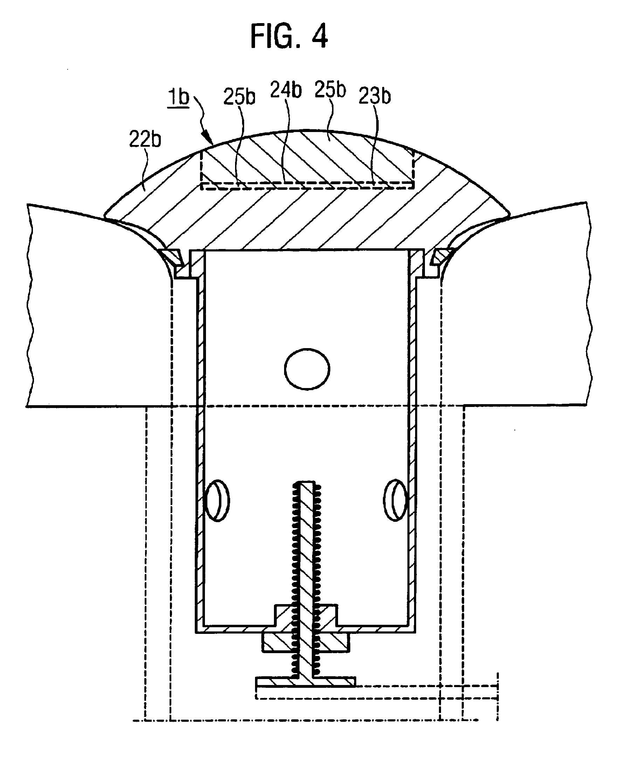

Wie vor allem auch Fig. 1 zeigt, bildet die dem Benutzer zugewandte Oberfläche 21 des Verschlussteiles einen balligen Verschlusskopf 22. Dieser Verschlusskopf 22 kann ganz oder teilweise durchsichtig sein, wie dies in Fig. 4 bis 6 dargestellt ist. Dies ist dann zweckmäßig, wenn gemäß dem in Fig. 4 dargestellten Ausführungsbeispiel ein Verschlusskopf 22b auf seiner dem Benutzer zugewandten Seite derart gestaltet ist, dass ein Informationsträger 23b sichtbar ist.

Als Informationsträger 23b dient entweder eine Fläche 24b oder ein in den Verschlusskopf 22b einlegbares Teil, das sich z. B. unterhalb eines durchsichtigen Einsatzes 25b befindet.

An die Form und Gestalt des Einsatzes 25b bzw. an ein beliebiges, dem Benutzer zugewandtes, mindestens teilweise durchsichtiges Abdeckteil sind keine Grenzen gesetzt.

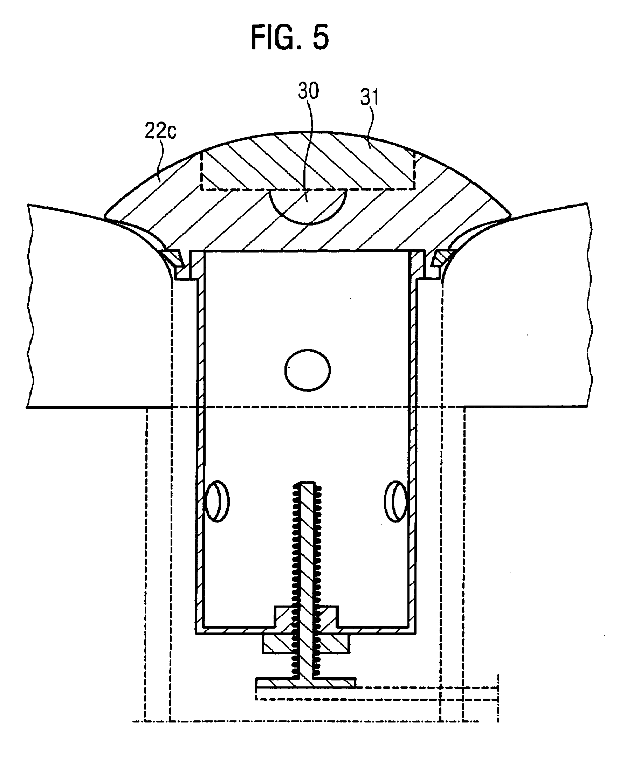

Grundsätzlich kann sich auch eine Lichtquelle 30c benutzerseitig im Verschlusskopf 22c unterhalb eines durchsichtigen Abdeckteiles 31c befinden, wie dies in Fig. 5 dargestellt ist.

Anstelle einer Lichtquelle 30c können grundsätzlich auch ein Temperaturfühler 32d und eine Temperaturanzeige 33d im Verschlusskopf 22b derart angeordnet sein, dass ein Benutzer die Angaben auf der Temperaturanzeige 33d durch ein durchsichtiges Teil 34d erkennen kann.

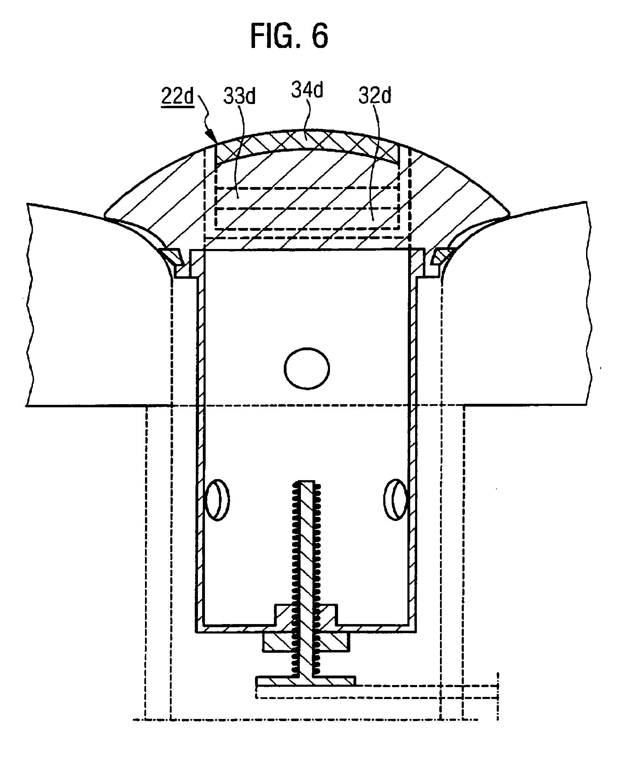

Grundsätzlich gleiche Teile des in Fig. 6 dargestellten Ausführungsbeispieles weisen dieselben Bezugszahlen wie die anderen Ausführungsformen und zusätzlich den Buchstabenindex d auf.

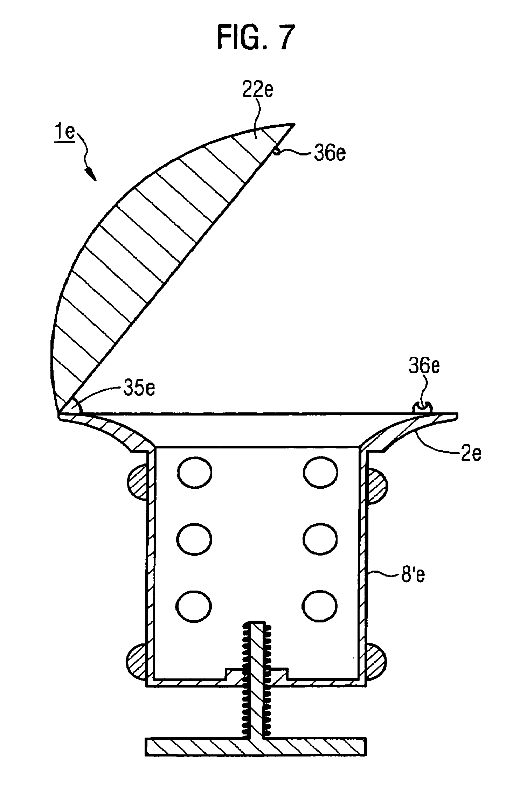

Ein letztes Ausführungsbeispiel von einem Verschlussstopfen 1e umfasst gemäß Fig. 7 einen Verschlusskopf 22e, der schwenkbar an dem den Behälter 8'e tragenden Verschlussteil 2e angeordnet ist. Auch bei diesem Ausführungsbeispiel gilt, dass gleiche Teile dieselben Bezugszahlen und zusätzlich den Buchstabenindex e aufweisen.

Zur schwenkbaren Befestigung ist ein Gelenk 35e vorgesehen, das den Verschlusskopf 22e mit dem Verschlussteil 2e verbindet. Eine Rasteinrichtung 36e hält den Verschlusskopf 22e, wenn er aus der geöffneten Stellung wieder in der Schließstellung verbleiben soll.

Die verschiedenen Ausführungsbeispiele lassen schließlich erkennen, dass mannigfache Abwandlung möglich sind, ohne von dem grundsätzlichen Erfindungsgedanken abzuweichen.

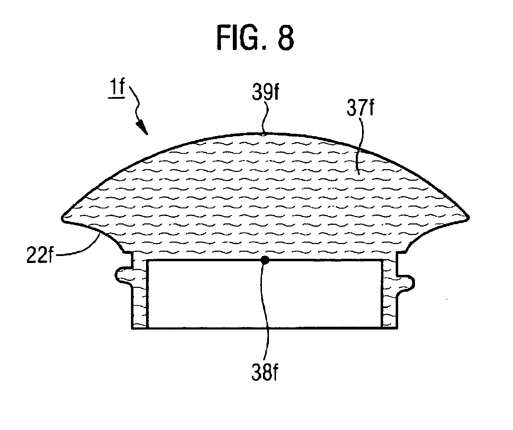

So zeigt Fig. 8 ein letztes Ausführungsbeispiel von einem Verschlussstopfen 1f, der zusammen mit einem in Fig. 8 nicht dargestellten Träger oder Behälter für eine hier interessierende Substanz oder auch ohne einen derartigen Behälter verwendbar ist. Dazu weist der Verschlussstopfen 1f einen Verschlusskopf 22f auf, der teilweise oder vollständig aus einem durchsichtigen Werkstoff besteht und eine Hohlkammer 37f zu Aufnahme einer flüssigen Substanz aufweist. Diese flüssige Substanz kann z. B. ein Duftstoff oder ein Parfüm sein. Bei dieser flüssigen Substanz kann es sich aber auch um einen flüssigen Reiniger bzw. um ein flüssiges Desinfektionsmittel handeln.

Zur Abgabe der flüssigen Substanz aus der Hohlkammer 37f im Verschlusskopf 22f dient eine Öffnung 38f, die jeweils nur in geringen Mengen, wie z. B. tropfenweise, bei einem entsprechenden Unterdruck in dem Raum unterhalb des Verschlusskopfes 22f Flüssigkeit abgibt. Derartige Öffnungen 38f sind grundsätzlich bekannt.

Fig. 8 zeigt zwar einen Verschlussstopfen 1f ohne zusätzlichen Träger 8 bzw. Behälter 8', doch versteht es sich, dass ein derartiger Träger oder Behälter auch zusätzlich vorgesehen sein kann und eine Substanz enthält, die sich mit der flüssigen Substanz in der Hohlkammer 37f ergänzt. So kann die Hohlkammer 37f einen Duftstoff aufnehmen, während in dem Behälter eine zum Reinigen dienende Substanz vorgesehen ist.

Da mindestens Teile der Oberfläche 39f bis zur Hohlkammer 37f aus einem durchsichtigen Werkstoff bestehen sollen, kann der Benutzer die noch vorhandene Menge des flüssigen Duftstoffes beobachten und bei Bedarf nachfüllen.

### Bezugszeichenliste

- 1, 1a, 1e, 1f: Verschlussstopfen
- 2, 2a, 2e: Verschlussteil
- 3, 3a: Dichtfläche / Dichtung
- 4: Rand der Ablauföffnung 5
- 5: Ablauföffnung
- 6: Becken / Behälter
- 7, 7a: Anschlag
- 8,8', 8'a, 8'e: Träger /Behälter
- 9, 9a: Substanz/Mittel
- 10: Boden
- 11, 11a: Behälterwand
- 12, 12', 12a, 12'a: Durchtrittsöffnungen
- 13: Behälterinneres
- 14: Raum
- 15: Rohr
- 16: Arm
- 17a: Distanzrippen
- 18a: Festkörper
- 19a: Längsbohrung
- 20a: Außenumfang
- 21: Oberfläche
- 22, 22b, 22c, 22e, 22f: Verschlusskopf
- 23b: Informationsträger
- 24b: Fläche
- 25b: Einsatz
- 30c: Lichtquelle
- 31 c: Abdeckteil
- 32e: Temperaturfühler
- 33d: Temperaturanzeige
- 34d: durchsichtiges Teil
- 35e: Gelenk
- 36e: Rasteinrichtung
- 37f: Hohlkammer
- 38f: Öffnungen
- 39f: Oberfläche

## Patentansprüche

1. Verschlussstopfen (1) mit einem mindestens eine Dichtfläche oder Dichtung (3) aufweisenden und am Rand (4) einer Ablauföffnung (5) eines Beckens (6), Behälters oder dergleichen, insbesondere eines Wasser- oder Waschbeckens, eines Spülbeckens, einer Badewanne, einer Duschwanne oder dergleichen unmittelbar anliegenden Verschlussteil (2, 2a) und einen ablaufseitig daran angeordneten von Wasser umströmten, mindestens teilweise offenen Träger (8), der im Bereich und/oder unterhalb der Ablauföffnung (5) mindestens eine Durchtrittsöffnung (12, 12', 12a, 12'a) für einen Flüssigkeits- und Substanzaustausch zwischen einem ihn außen umgebenden Raum (14) und seinem Inneren aufweist,
**dadurch gekennzeichnet, dass** der Träger (8) für die Aufnahme einer sich im Wasser allmählich verbrauchenden Substanz (9) oder eines Mittels geeignet ist.

2. Verschlussstopfen nach Anspruch 1,
**dadurch gekennzeichnet, dass**
der Träger (8) ein lösbarer und mit der sich verbrauchenden Substanz (9)/ Mittel nachfüllbarer Behälter (8') ist.

3. Verschlussstopfen nach Anspruch 1 und 2,
**dadurch gekennzeichnet, dass**
der Behälter (8') am Verschlussteil (2) mit Hilfe einer Steckverbindung befestigbar ist.

4. Verschlussstopfen nach Anspruch 2 oder 3,
**dadurch gekennzeichnet, dass**
ein Verschlusskopf schwenkbar an dem den Behälter (8') tragenden Verschlussteil (2) angeordnet ist.

5. Verschlussstopfen nach einem der Ansprüche 1 bis 4,
**dadurch gekennzeichnet, dass**
das Verschlussteil (2) mindestens teilweise durchsichtig ist.

6. Verschlussstopfen nach mindestens einem der vorhergehenden Ansprüche,
**dadurch gekennzeichnet, dass**
das Verschlussteil (2) auf seiner dem Benutzer zugewandten Seite derart gestaltet ist, dass ein Informationsträger (23b) sichtbar ist.

7. Verschlussstopfen nach einem der Ansprüche 1 bis 6,
**dadurch gekennzeichnet, dass**
benutzerseitig eine Lichtquelle (30c) und/oder ein Temperaturfühler und eine Temperaturanzeige im Verschlussteil (2) angeordnet sind.

8. Verschlussstopfen nach mindestens einem der vorhergehenden Ansprüche,
**dadurch gekennzeichnet, dass**
mindestens ein Langloch (12a) in einer Behälterwand (11) als Durchtrittsöffnung und/oder dass Distanzrippen (17a) außen am Behälter (7'a) angeordnet sind.

9. Verschlussstopfen nach mindestens einem der vorhergehenden Ansprüche,
**dadurch gekennzeichnet, dass**
zusätzlich zu dem Träger (8) eine Hohlkammer (37f) und eine Abgabeöffnung (38f) für eine flüssige Substanz vorgesehen sind.

## Claims

1. A drain plug (1) having a closing piece (2, 2a) including at least one sealing face or sealing (3) and being immediately adjacent to the edge (4) of a drain opening (5) of a basin (6), container or the like, in particular of a water or wash basin, kitchen sink, bath tub, shower tub or the like, and having a carrier (8) which is arranged on its drain side, around which the water flows, which is at least partially open, and which includes in the area of, and/or below, the drain opening (5) at least one passage opening (12, 12', 12a, 12'a) for an exchange of liquid and matter between a space (14) externally surrounding it and its inside,
**characterized in that**
the carrier (8) is suited for receiving a substance (9) or product which will gradually be consumed in the water.

2. The drain plug (1) of claim 1,
**characterized in that**
the carrier (8) is a detachable container (8') which can be refilled with the substance (9)/product being consumed.

3. The drain plug (1) of claims 1 and 2,
**characterized in that**
the container (8') is fastened to the closing piece (2) by means of a plug-in connection.

4. The drain plug (1) of claim 2 or 3,
**characterized in that**
a closing head is pivotably arranged on the closing piece (2) carrying the container (8').

5. The drain plug (1) of any of claims 1 to 4,
**characterized in that**
the closing piece (2) is at least partially transparent.

6. The drain plug (1) of at least one of the preceding claims,
**characterized in that**
the closing piece (2) is designed, on its side facing towards the user, in such a way that an information carrier (23b) is visible.

7. The drain plug (1) of any of claims 1 to 6,
**characterized in that**
on the user side, a light source (30c) and/or a temperature probe and a temperature display are arranged in the closing piece (2).

8. The drain plug (1) of at least one of the preceding claims,
**characterized in that**
at least one oblong hole (12a) is arranged in one container wall (11) as a passage opening and/or that spacer ribs (17a) are arranged on the outside of the container (8'a).

9. The drain plug (1) of at least one of the preceding claims,
**characterized in that**
in addition to the carrier (8), a hollow chamber (37f) and a discharge opening (38f) for a liquid substance are provided.

## Revendications

1. Bonde de lavabo (1) ayant une pièce de fermeture (2, 2a) comprenant au moins une surface d'étanchéité ou un joint (3) et étant immédiatement adjacente au bord (4) d'une ouverture d'égout (5) d'un bassin (6), d'un récipient ou semblable, en particulier d'un bassin d'eau ou d'un lavabo, d'un évier, d'une baignoire, d'une cuve à douche ou semblable, et ayant un support (8) qui est disposé à son côté d'égout, autour duquel l'eau s'écoule, qui est au moins partiellement ouvert et qui comprend dans dans la zone et/ou au-dessous de l'ouverture d'égout (5) au moins une ouverture de passage (12, 12', 12a, 12'a) pour un échange de liquide et de matière entre un espace (14) l'entourant à l'extérieur et son intérieur,
**caractérisé en ce**
**que** le support (8) est approprié à recevoir une substance (9) ou un produit qui sera graduellement consommé dans l'eau.

2. Bonde de lavabo (1) selon la revendication 1,
**caractérisé en ce**
**que** le support (8) est un récipient (8') détachable qui peut être rempli de la substance (9)/du produit étant consommé.

3. Bonde de lavabo (1) selon les revendications 1 et 2,
**caractérisé en ce**
**que** le récipient (8') est fixé à la pièce de fermeture (2) au moyen d'un raccordement à fiche.

4. Bonde de lavabo (1) selon la revendication 2 ou 3,
**caractérisé en ce**
**qu'**une tête de fermeture est disposé de manière pivotante à la pièce de fermeture (2) portant le récipient (8').

5. Bonde de lavabo (1) selon l'une quelconque des revendications 1 à 4,
**caractérisé en ce**
**que** la pièce de fermeture (2) est au moins partiellement transparente.

6. Bonde de lavabo (1) selon au moins une des revendications précédentes,
**caractérisé en ce**
**que** la pièce de fermeture (2) est formée, de son côté en regard de l'utilisateur, de façon qu'un support d'information (23b) est visible.

7. Bonde de lavabo (1) selon l'une quelconque des revendications 1 à 6,
**caractérisé en ce**
**que**, du côté d'utilisateur, une source de lumière (30c) et/ou une sonde de température et un affichage de température sont disposés dans la pièce de fermeture (2).

8. Bonde de lavabo (1) selon au moins une des revendications précédentes,
**caractérisé en ce**
**qu'**au moins un trou oblong (12a) est disposé dans un mur du récipient (11) comme ouverture de passage et/ou que des nervures d'espacement (17a) sont disposées à l'extérieur du récipient (8'a).

9. Bonde de lavabo (1) selon au moins une des revendications précédentes,
**caractérisé en ce**
**que**, en plus du support (8), une chambre creuse (37f) et une ouverture de décharge (38f) pour une substance liquide sont prévues.
